**Europäisches Patentamt**

**European Patent Office**

**Office européen des brevets**

(11) Veröffentlichungsnummer : **0 175 227 B1**

# (12) EUROPÄISCHE PATENTSCHRIFT

(45) Veröffentlichungstag der Patentschrift : **11.11.87**

(21) Anmeldenummer : **85111253.2**

(22) Anmeldetag : **06.09.85**

(51) Int. Cl.⁴ : **C 07 C143/38**

(54) Verfahren zur Herstellung von Alkali- und Erdalkalisalzen der Benzaldehyd-2,4-disulfonsäure.

(30) Priorität : **17.09.84 DE 3434038**

(43) Veröffentlichungstag der Anmeldung :
**26.03.86 Patentblatt 86/13**

(45) Bekanntmachung des Hinweises auf die Patenterteilung : **11.11.87 Patentblatt 87/46**

(84) Benannte Vertragsstaaten :
**CH DE FR GB IT LI**

(56) Entgegenhaltungen :
**DE-C-   32 238**

(73) Patentinhaber : **HOECHST AKTIENGESELLSCHAFT**
**Postfach 80 03 20**
**D-6230 Frankfurt am Main 80 (DE)**

(72) Erfinder : **Metz, Hans Joachim, Dr.**
**Reiterweg 8**
**D-6148 Heppenheim (DE)**

## Beschreibung

Die Erfindung betrifft ein Verfahren zur Herstellung von Alkali- und Erdalkalisalzen der Benzaldehyd-2,4-disulfonsäure aus 2,4-Dichlorbenzalchlorid.

Benzaldehyd-2,4-disulfonsäure und ihre Salze stellen wertvolle technische Produkte dar und finden beispielsweise Verwendung in der galvanischen Industrie oder bei der Produktion von Triphenylmethanfarbstoffen.

Für die Herstellung des Dinatriumsalzes der Benzaldehyd-2,4-disulfonsäure aus 2,4-Dichlorbenzalchlorid ist bisher nur eine zweistufige Verfahrensweise bekannt.

1. Herstellung von 2,4-Dichlorbenzaldehyd aus 2,4-Dichlorbenzalchlorid.

2. Umsetzung des erhaltenen 2,4-Dichlorbenzaldehyds mit Natriumsulfit und/oder -hydrogensulfit.

Nach dem Stand der Technik sind für die erste Stufe mehrere Methoden verfügbar. Beispielsweise wird 2,4-Dichlorbenzalchlorid mit einem Gemisch aus konzentrierter Schwefelsäure und Oleum bei 40-50 °C in die Bisulfitverbindung des Aldehyds überführt der Aldehyd mit Sodalösung freigesetzt und durch Wasserdampfdestillation gewonnen (siehe DRP 32238 und Liebigs Ann. 260, 68).

Auch die direkte Hydrolyse von 2,4-Dichlorbenzalchlorid mit Zinkoxid als Katalysator bei 110-140 °C sei noch erwähnt (s. japanische Offenlegungsschrift Nr. 77/25733).

Der Stand der Technik der zweiten Stufe ist durch die deutschen Reichspatente DRP 88952, DRP 91818 und DRP 98321 gegeben.

So wird im DRP 88952 die Umsetzung von 2-Chlorbenzaldehyd mit einer wäßrigen Natriumhydrogensulfit-Lösung bei Reaktionstemperaturen von 190-200 °C und Reaktionszeiten von 8 h zum Natriumsalz der Benzaldehyd-2-sulfonsäure beschrieben. Analog erhält man gemäß dem DRP 91818 aus 2,5-Dichlorbenzaldehyd das Natriumsalz der 5-Chlorbenzaldehyd-2-sulfonsäure. Schließlich wird im DRP 98321 ein analoges Verfahren zur Gewinnung einer wäßrigen Lösung des Dinatriumsalzes der Benzaldehyd-2,4-disulfonsäure aus 2,4-Dichlorbenzaldehyd bei Reaktionstemperaturen von 190-200 °C und Reaktionszeiten von 9-10 h beschrieben. Die Substanz wird dabei jedoch nicht isoliert, auch fehlen Angaben zur Ausbeute. Bei der Durchführung der Reaktion unter den im Beispiel des DRP 98321 genannten Bedingungen entsteht ein großer Anteil an Nebenprodukten. Man erhält dunkel gefärbte, heterogene Reaktionsmischungen, aus denen nur mit erheblichem Aufwand stark verunreinigtes Produkt mit einer Ausbeute von 30-60 % d. Th. isoliert werden kann.

Das Gesamtverfahren über zwei Stufen ist mit den Nachteilen der geringen Ausbeute, schlechten Reproduzierbarkeit, und trotz Zwischenisolierung, der geringen Reinheit des gewünschten Produkts behaftet.

Daher bestand die Aufgabe, ein entsprechendes Verfahren zu entwickeln, das in reproduzierbarer Weise das gewünschte Produkt in wirtschaftlichen Ausbeuten liefert.

Es wurde nun ein Verfahren zur Herstellung eines Alkali- oder Erdalkalisalzes der Benzaldehyd-2,4-disulfonsäure aus 2,4-Dichlorbenzalchlorid gefunden, dadurch gekennzeichnet, daß 2,4-Dichlorbenzalchlorid mit einem Alkali- oder Erdalkalisulfit und/oder -hydrogensulfit in Gegenwart von Wasser und säurebindenden Substanzen, vorzugsweise bei pH 7-12, bei einer Temperatur von 140-180 °C, vorzugsweise 160-170 °C, umgesetzt wird.

Substituierte Benzalchloride und Benzaldehyde sind reaktive Verbindungen, die insbesondere im alkalischen Milieu den verschiedensten Reaktionen unterliegen, z. B. Kondensationen oder der Cannizzaro-Reaktion. Es ist daher ausgesprochen überraschend, daß trotz der vielfältigen Reaktionsmöglichkeiten im System 2,4-Dichlorbenzalchlorid/2,4-Dichlorbenzaldehyd/Sulfit (bzw. Hydrogensulfit)/Wasser/säurebindende Substanz ganz überwiegend das gewünschte Produkt gebildet wird. Weiter wurde gefunden, daß entgegen der Lehre der oben zitierten Patente zur Reaktion mit Sulfit die optimale Reaktionstemperatur unter 180 °C, vorzugsweise bei 160-170 °C liegt und daß trotz der niedrigeren Temperatur eine Reaktionszeit von weniger als 7 h, vorzugsweise von nur 2-4 h, ausreicht.

Unter den erfindungsgemäßen Reaktionsbedingungen wird die Aldehydgruppe gebildet und werden mit Hilfe des Sulfits die Sulfonsäurereste eingeführt. Die bei der Bildung der Aldehydgruppe freigesetzte Salzsäure wird durch die im Reaktionsgemisch vorhandene Puffersubstanz neutralisiert. Dafür geeignet ist beispielsweise Natrium oder Kaliumcarbonat, Natrium- oder Kaliumhydrogenkarbonat, Magnesium- oder Calciumkarbonat, Magnesium- der Calciumhydrogenkarbonat, Calciumhydroxid bzw. Kalkmilch, Natrium- oder Kaliumphosphat oder überschüssiges Sulfit.

Es wird zweckmäßig so verfahren, daß man 2,4-Dichlorbenzalchlorid zusammen mit der Puffersubstanz und einer wäßrigen Lösung des Sulfits und/oder Hydrogensulfits unter Rühren in einem geschlossenen Gefäß erhitzt. Als Sulfite und Hydrogensulfite kommen besonders die des Natriums und Kaliums in Betracht.

Das Kation des Sulfits sollte mit dem des Hydrogensulfits und dem des als Puffersubstanz verwendeten Salzes übereinstimmen, um ein einheitliches Produkt zu erhalten.

Das Sulfit und/oder Hydrogensulfit wird vorteilhaft in einer Menge von 2-2,5 mol, vorzugsweise 2,05-2,15 mol, pro mol 2,4-Dichlorbenzalchlorid eingesetzt.

Das geeignete Massenverhältnis Sulfit und/oder Hydrogensulfit zu Wasser hängt teilweise von dessen Löslichkeit ab. Im Falle von Natriumsulfit liegt das Massenverhältnis vorzugsweise bei 0,11-0,33, insbesondere bei 0,22-0,25.

Der pH-Wert der Reaktionsmischung soll vorzugsweise zwischen den Werten 7 und 12 liegen.

Zur Isolierung des Reaktionsproduktes destilliert man zweckmäßig einen Teil des Wassers ab und läßt das Produkt unter Kühlung auskristallisieren.

Überschüssiges Sulfit und/oder Hydrogensulfit kann nach üblichen Methoden entfernt werden. Bevorzugt genannt sei die Oxidation mit wäßriger Natriumhypochlorit-Lösung im Anschluß an die Kristallisation des Produkts. Das Reaktionsprodukt kann aus der Mutterlauge in einfacher Weise durch Zentrifugieren abgetrennt werden.

Die Vorteile der neuen Arbeitsweise gegenüber dem Stand der Technik liegen in dem geringeren Aufwand durch das einstufige Verfahren, in der Reproduzierbarkeit, hohen Ausbeute, Energie-Ersparnis infolge stark verkürzter Reaktionszeit und der wesentlich gesteigerten Raum-Zeit-Ausbeute begründet.

Beispiel 1

230 g (1 mol) 2,4-Dichlorbenzalchlorid werden in einem geschlossenen Gefäß zusammen mit einer Lösung von 260 g (2,06 mol) Natriumsulfit und 85 g (0,8 mol) Natriumkarbonat in 1 040 g Wasser 3,5 h auf 170 °C erhitzt. Nach Einengen auf 70 Gew.-% der Einwaage und Oxidation mit 140 g Natriumhypochlorit-Lösung (13 Gew.-% NaOCl) erhält man durch Zentrifugieren 307 g Produkt mit einem Gehalt von 74,4 Gew.-% an Benzaldehyd-2,4-disulfonsäure-di-natriumsalz, = 73,5 % d. Th.

Beispiel 2

230 g (1 mol) 2,4-Dichlorbenzalchlorid werden in einem geschlossenen Gefäß zusammen mit einer Lösung von 260 g (2,06 mol) Natriumsulfit und 180 g (2,14 mol) Natriumhydrogencarbonat in 1 045 g Wasser 3,5 h auf 170 °C erhitzt. Nach Einengen auf 70 % der Einwaage und Oxidation mit 140 g Natriumhypochloritlösung (13 Gew.-% NaOCl) erhält man durch Zentrifugieren 313 g Produkt mit einem Gehalt von 74,9 % an Benzaldehyd-2,4-di-sulfonsäure-dinatriumsalz ≙ 75,6 % d. Th.

**Patentansprüche**

1. Verfahren zur Herstellung eines Alkali- oder Erdalkalisalzes der Benzaldehyd-2,4-disulfonsäure aus 2,4-Dichlorbenzalchlorid, dadurch gekennzeichnet, daß 2,4-Dichlorbenzalchlorid mit einem Alkali- oder Erdalkalisulfit und/oder -hydrogensulfit in Gegenwart von Wasser und säurebindenden Substanzen bei einer Temperatur von 140-180 °C umgesetzt wird.

2. Verfahren nach Anspruch 1, dadurch gekennzeichnet, daß die Reaktion bei 160-170 °C durchgeführt wird.

3. Verfahren nach Anspruch 1 oder 2, dadurch gekennzeichnet, daß die Reaktion bei pH 7-12 durchgeführt wird.

4. Verfahren nach einem der Ansprüche 1 bis 3, dadurch gekennzeichnet, daß pro mol Ausgangsstoff 2-2,5 mol Sulfit und/oder Hydrogensulfit in Form einer wäßrigen Lösung eingesetzt wird.

5. Verfahren nach einem der Ansprüche 1 bis 4, dadurch gekennzeichnet, daß als säurebindende Substanz Natriumkarbonat und/oder Natriumhydrogencarbonat verwendet wird.

6. Verfahren nach einem der Ansprüche 1 bis 5, dadurch gekennzeichnet, daß eine wäßrige Natriumsulfit-Lösung mit einem Massenverhältnis Natriumsulfit/zu Wasser von 0,11 bis 0,33 eingesetzt wird.

**Claims**

1. Process for the preparation of an alkali metal or alkaline earth metal salt of benzaldehyde-2,4-disulfonic acid from 2,4-dichlorobenzal chloride, characterized in that 2,4-dichlorobenzal chloride is reacted with an alkali metal or alkaline earth metal sulfite and/or hydrogensulfite in the presence of water and acid-bonding substances at a temperature of 140-180 °C.

2. Process as claimed in claim 1, characterized in that the reaction is carried out at 160-170 °C.

3. Process as claimed in claim 1 or 2, characterized in that the reaction is carried out at a pH of 7-12.

4. Process as claimed in one of the claims 1 to 3, characterized in that 2-2.5 moles of sulfite and/or hydrogensulfite in the form of an aqueous solution are used per mole of starting material.

5. Process as claimed in one of the claims 1 to 4, characterized in that sodium carbonate and/or sodium hydrogencarbonate is used as the acid-bonding substance.

6. Process as claimed in one of the claims 1 to 5, characterized in that an aqueous sodium sulfite solution is used with a mass ratio of sodium sulfite to water of 0.11 to 0.33.

**Revendications**

1. Procédé de préparation d'un sel de métal alcalin ou de métal alcalino-terreux de l'acide formyl-1 benzènedisulfonique-2,4 à partir du chlorure de dichloro-2,4 benzylidène, procédé caractérisé en ce qu'on fait réagir le dichlorure de dichloro-2,4 benzylidène avec un sulfite et/ou un hydrogénosulfite de métal alcalin ou de métal alcalino-terreux, en présence d'eau et d'accepteurs d'acides, à une température de 140 à 180 °C.

2. Procédé selon la revendication 1 caractérisé en ce qu'on effectue la réaction à une température de 160 à 170 °C.

3. Procédé selon l'une des revendications 1 et 2, caractérisé en ce qu'on effectue la réaction à un pH de 7 à 12.

4. Procédé selon l'une quelconque des revendications 1 à 3, caractérisé en ce qu'on met en jeu, par mole du corps de départ, de 2 à 2,5 mol du sulfite et/ou de l'hydrogénosulfite sous la forme d'une solution aqueuse.

5. Procédé selon l'une quelconque des revendications 1 à 4, caractérisé en ce qu'on utilise, comme accepteurs d'acides, le carbonate de sodium et/ou l'hydrogénocarbonate de sodium.

6. Procédé selon l'une quelconque des revendications 1 à 5, caractérisé en ce qu'on utilise une solution aqueuse de sulfite de sodium avec un rapport massique du sulfite de sodium à l'eau compris entre 0,11 et 0,33.